# EUROPEAN PATENT APPLICATION

(11) **EP 4 538 282 A1**
(43) Date of publication of application: **16.04.2025**
(21) Application number: 23306797.4
(22) Date of filing: 13.10.2023
(51) Int. Cl.: C07K 11/02

(54) **SYNTHESIS OF EMODEPSIDE**

(71) Applicant: Vetoquinol SA, 70200 Lure (FR)
(72) Inventor: MORRISON, Alec, 4058 Basel (CH); LAGOUTTE, Roman, 4058 Basel (CH)
(74) Representative: Novagraaf Technologies

(57) **Abstract**

The present invention relates to a three-step synthesis of emodepside of formula (I) from PF 1022 A:

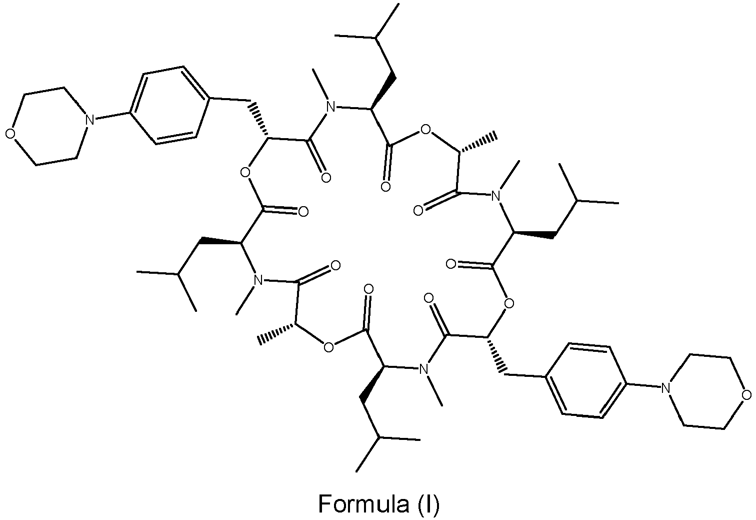

## Description

### Technical field

The present invention belongs to the field of antiparasitics and more particularly to emodepside, which is generally used to prevent and/or treat endoparasites. It also belongs to the class of drugs known as octadepsipeptides.

The prevent invention relates to a process for the synthesis of emodepside, to the reaction intermediates of said synthesis process and the use of said reaction intermediates in a synthesis process of emodepside.

In the description below, references between **[ ]** refer to the list of references at the end of the examples.

### Technical background

Emodepside is an anthelmintic drug that is effective against a number of gastrointestinal nematodes and belongs to the class of drugs known as the octadepsipeptides, a relatively new class of anthelmintic (research into these compounds began in the early 1990s), which are suspected to achieve their antiparasitic effect by a novel mechanism of action due to their ability to kill nematodes resistant to other anthelmintics. Its formula (formula (I)) is as follows:

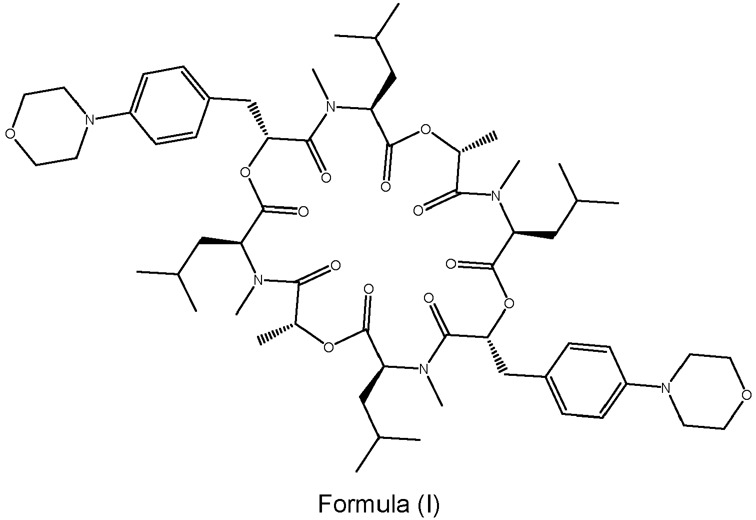

Several synthesis processes of emodepside have been described in the literature and patent applications.

For example, the patent WO 2019091975 describes the synthesis of Emodepside by macrolactamisation from the open form. This patent mentions more specifically the macrocyclisation of substituted benzyl derivatives and the p-morpholinobenzyl derivatives, with propanephosphonic acid anhydride/N,N-diisopropyléthylamine (T3P/DIPEA) or benzotriazol-1-yloxytripyrrolidinophosphonium hexafluorophosphate/ N,N-diisopropyléthylamine (PyBOP/DIPEA) as preferred cyclisation reagent. There is also the patent WO 2019040585 which describes the hemisynthesis of Emodepside from PF-1022A:

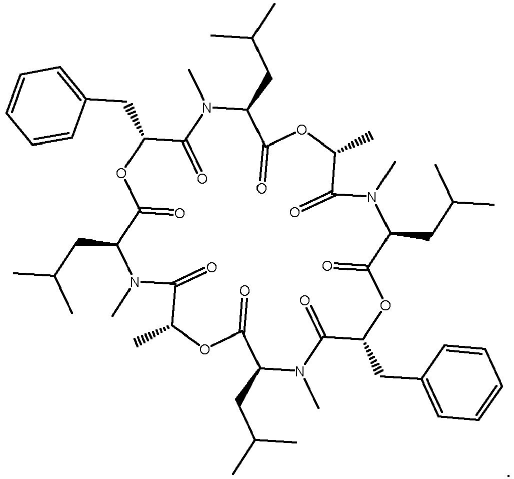

The patent claims more specifically the modification of PF-1022A and elaboration into Emodepside by the series of reactions:
- Nitration and reduction to the aniline
- N,N bis-alkylation or reductive amination with the required ethereal bis-electrophile (Cl, Br, OTs, OMs, aldehyde) to build the morpholine ring.

The patent WO 2019040589 also describes the hemisynthesis of Emodepside from PF-1022A.

Despite great accomplishments in the search for the synthesis of emodepside, some challenges remain to be addressed. For instance, the synthesis strategies remain limited, expensive while presenting reaction conditions that can be problematic for scale-up, as well as a low overall yield.

### Detailed description of the invention

The inventors have developed a new process comprising several steps for the synthesis of the emodepside from its precursor PF-1022A.

The inventors surprisingly found out a synthesis process allowing to access to Emodepside from PF-1022A in few steps, while having a great overall yield and in adequacy with a practice of green chemistry. Moreover, this process is a chromatography-free synthesis, and it is precious metal-free.

The invention thus relates in a first aspect to a process for the synthesis of emodepside comprising a step of reacting morpholine with a reaction intermediate of formula (II): 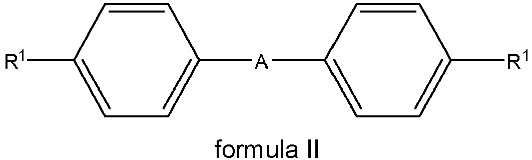 in which,
- R¹ is an aliphatic or aromatic borate group comprising 6 or fewer carbon atoms;
- A is 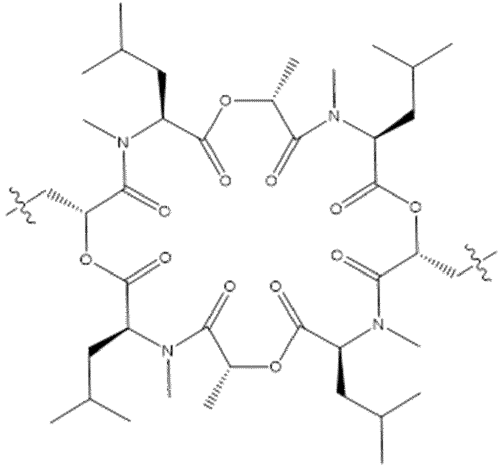 in the presence of a catalyst in at least one organic solvent.

Advantageously, R¹ may be selected from -B(OH)2,

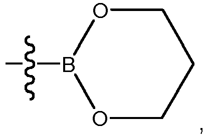

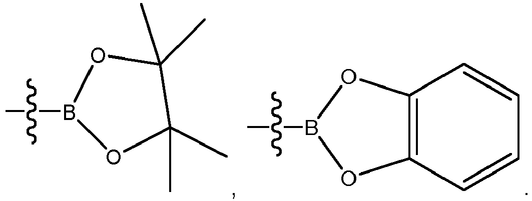

Advantageously, the catalyst used for the synthesis of emodepside may be selected from Cu(OAc)₂, Cu[MeCN]₄BF₄, Cu(DMAP₄I)I, CuI, Cu₂O and [Cu₂(TMEDA)₂]Cl₂.

It is meant by "DMAP", the group 4-dimethylaminopyridine.

It is meant by "TMEDA", the group tetramethylethylenediamine.

Advantageously, the at least one organic solvent use for the synthesis of emodepside may be selected from acetonitrile, N-methyl pyrrolidone, toluene, dioxane, dichloromethane (DCM), dimethylformamide (DMF) and mixtures thereof.

Advantageously, the reaction for the synthesis of emodepside may be carried out in the presence of an acid or a base catalyst, preferably selected from K₂CO₃, triethyl amine (TEA), morpholine, tetramethylene diamine (TMEDA), 2,6-lutidine, Na₂CO₃ and B(OH)₃.

Advantageously, the reaction for the synthesis of emodepside may be carried out at a temperature from 15 °C to 80 °C, preferably from 15 °C to 60 °C and more preferably at room temperature.

It is meant by "room temperature" a temperature from 15 °C to 25 °C, preferably 20 °C.

Advantageously, the reaction for the synthesis of emodepside may be carried out over a period of 1 to 64 hours, preferably 2 to 16 hours and more preferably 6 hours.

Advantageously, the process for the synthesis of emodepside may further comprise a work-up step, preferably a step of filtration, washing, acid-base extraction and/or concentration.

It is meant by "filtration", a step according to one of the following methods: the filtration method involving the passage of a liquid mixture through a porous medium; the vacuum filtration method utilizing a vacuum system to accelerate the filtration process; the centrifugal filtration method employing centrifugal force to separate solid particles; the gravity filtration method relying on gravity to drive a liquid through a filter medium; the microfiltration method utilizing a microporous membrane or filter; the ultrafiltration method using a semipermeable membrane to separate larger molecules; and filtration under elevated pressure, applying pressure for increased separation efficiency.

It is meant by "washing", a step according to one of the following methods: the washing method involving the treatment of a product with a solvent to remove impurities; the liquid-liquid extraction method involving the partitioning of product and impurities between two immiscible liquid phases; the solid-phase extraction method utilizing a solid adsorbent material to selectively bind and remove impurities; the recrystallization method involving dissolving a product in a suitable solvent and allowing it to slowly crystallize, leaving impurities behind; and the washing under ultrasonic conditions, applying ultrasonic waves to facilitate the removal of impurities from a product.

It is meant by "acid-base extraction", a step according to one of the following methods: the acid-base extraction method involving the treatment of a mixture with acidic and basic solutions to selectively extract desired components; the liquid-liquid extraction method utilizing the differential solubility of compounds in acidic and basic solutions to partition them between immiscible liquid phases; the solid-phase extraction method utilizing a solid adsorbent material functionalized with acidic or basic groups to selectively bind and extract compounds; and the continuous flow extraction method utilizing a continuous flow system for efficient and rapid extraction.

It is meant by "concentration", a step according to one of the following methods: the concentration method involving the removal of volatile components under vacuum to increase the concentration of the desired components; the freeze-drying method where a sample is frozen and subjected to low pressure, causing the frozen solvent to sublime and leave behind a concentrated product; the nitrogen blow-down method where an inert gas such as nitrogen is passed over a sample to facilitate evaporation and concentration; and the solvent removal method using techniques such as gentle heating, vacuum, or a combination thereof to remove a solvent and concentrate a sample.

The invention relates in a second aspect to a process for the synthesis of a reaction intermediate of general formula (II) comprising a step of reacting a compound comprising an aliphatic or aromatic borate comprising 6 or fewer carbon atoms with a reaction intermediate of formula (III): 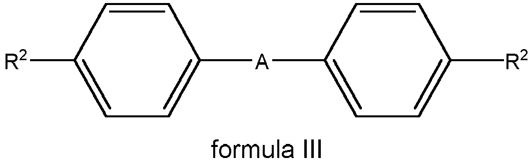 in which
- R² is a substituted or unsubstituted thianthrene group;
- A is defined as mentioned above
in the presence of at least one solvent.

Advantageously, the substituted or unsubstituted thianthrene group may be selected from 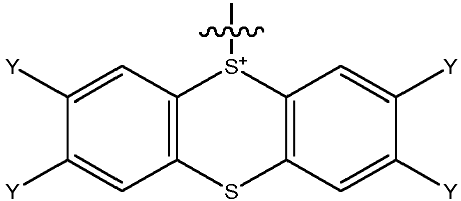 in which Y is selected from H and a halo group. Preferably, Y may be a halo group, more preferably a halo group selected from fluoro, chloro and bromo group.

Advantageously, the aliphatic or aromatic borate comprising 6 or fewer carbon atoms used for the synthesis of a reaction intermediate of general formula (II) may be selected from 2,2-Bi-1,3,2-dioxaborinane, bis(pinacolato)boron and bis(catecholato)boron, -B₂(OH)₄.

Advantageously, the solvent used for the synthesis of a reaction intermediate of general formula (II) may be selected from acetonitrile, acetone, ethanol, ethylene glycol, water, dimethylacetamide (DMAc), DMF and mixtures thereof.

Advantageously, the reaction for the synthesis of a reaction intermediate of general formula (II) may be carried out at a temperature from 15 to 80 °C, preferably above 20°C and more preferably above 40 °C.

Advantageously, the reaction for the synthesis of a reaction intermediate of general formula (II) may be carried out over a period of 4 to 72 hours, preferably more than 16 hours and more preferably up to 72 hours in batch mode or 3 to 20 hours in flow mode.

Advantageously, the reaction for the synthesis of a reaction intermediate of general formula (II) may further comprise a work-up step, preferably a step of cooling, pouring into aqueous acid, filtering, washing after 6 different solvents/solutions, drying.

It is meant by "cooling", a step according to one of the following methods: the cooling method involving the reduction of temperature to facilitate the precipitation, crystallization, or solidification of desired components from a solution or mixture; the evaporative cooling method where cooling is achieved through the evaporation of a volatile liquid, which absorbs heat from the surroundings; the cooling by circulation method where a coolant, such as chilled water or refrigerant, is circulated through a heat exchanger to lower the temperature of a sample; the cooling by controlled refrigeration method using precise temperature control systems or refrigeration units to maintain desired cooling conditions; and the cooling by indirect heat transfer method where a sample is cooled by indirect contact with a cold surface or coolant.

It is meant by "pouring into aqueous acid", a step according to one of the following methods: the pouring into aqueous acid method involving the addition of a solution or mixture into an aqueous solution of an acid, typically mineral acids such as hydrochloric acid, sulfuric acid, or nitric acid; the acidification method where the pH of a solution or mixture is lowered by adding acid to create an acidic environment; the acid quenching method involving the rapid addition of an acid to terminate a reaction or deactivate reactive species; the acid extraction method where a target compound is selectively extracted into an acidic aqueous phase to separate it from other components; the acid hydrolysis method where a substance is subjected to hydrolysis under acidic conditions to break down chemical bonds; the acid precipitation method where an acid is added to a solution to cause the precipitation of a desired compound; the acid treatment method where a material is treated with acid to modify its properties or remove impurities; the acid washing method where a solid or liquid is washed with an acid to remove contaminants or impurities; and the acid neutralization method where an acid is added to neutralize a basic solution or compound.

The definition of "filtering" mentioned above also applies here.

The definition of "washing" mentioned above also applies here.

It is meant by "drying", a step according to one of the following methods: the drying method involving the removal of moisture or solvents from a substance or solution to obtain a dry product; the vacuum drying method where reduced pressure is applied to accelerate the evaporation of moisture or solvents; the oven drying method where a substance is subjected to elevated temperatures in an oven to promote evaporation; the freeze-drying method where a substance is frozen and subjected to low pressure, causing the frozen solvent to sublime and leave behind a dry product; the air drying method where a substance is exposed to air at ambient conditions to allow moisture or solvents to evaporate over time; the drying under inert gas method where a substance is dried in an atmosphere of inert gas, such as nitrogen or argon, to prevent oxidation or moisture absorption; the spray drying method where a solution or suspension is sprayed into a hot drying chamber, allowing rapid evaporation and drying; and the drying by filtration method where a solid or semi-solid substance is placed on a porous medium, such as filter paper, to facilitate the drying process.

Advantageously, the reaction for the synthesis of the reaction intermediate of general formula (II) may further comprise the following sub-steps:
a) mixing the compound comprising an aliphatic or aromatic borate comprising 6 or fewer carbon atoms with the reaction intermediate of formula (III);
b) optional introduction of a photocatalyst selected from Tris(2,2'-bipyridine)ruthenium(II) hexafluorophosphate (Ru(bpy)₃(PF₆)₂) and 2,4,5,6-tetra(carbazol-9-yl)benzene-1,3-dicarbonitrile (4czIPN);
c) irradiation of a loop of polyethylene (PE) tubing with a LED light;
d) recirculating the solution obtained in sub-step c) in the loop; and
the reaction being executed in a batch mode or a flow mode.

The use of the photocatalyst is made according to a method involving the introduction of a photosensitive substance or material that can absorb light and undergo photoexcitation, initiating or promoting a specific reaction; the photocatalytic oxidation method where a photocatalyst is employed to facilitate the oxidation of a substrate or compound using light as the driving force; and/or the photocatalytic reduction method where a photocatalyst is utilized to promote the reduction of a substrate or compound using light as the energy source.

It is meant by "irradiation", a step according to one of the following methods: the irradiation method involving the exposure of a substance or reaction mixture to light, typically in the visible or ultraviolet range, to initiate or promote a specific reaction; the photochemical reaction method where a chemical transformation occurs upon absorption of light by the reactants, leading to the formation of desired products; the photooxidation method where light energy is used to initiate oxidation reactions; and the photoreduction method where light energy is utilized to drive reduction reactions, resulting in the formation of reduced products.

It is meant by "LED light", the utilization of a light-emitting diode (LED) that emits light at specific wavelengths, including but not limited to blue light (450-500 nm), green light (520-565 nm), red light (620-750 nm), ultraviolet (UV) light (200-400 nm), or other wavelengths within the LED spectrum, to initiate or promote a specific reaction. Preferably, the LED light is a Blue LED light emitting wavelengths comprise from 450 to 500 nm.

It is meant by "recirculating" a step according to one of the following methods: the recirculating method involving the continuous circulation or recycling of a reaction mixture or solvent through a closed-loop system in flow chemistry; the continuous flow method where the reaction mixture or solvent is continuously pumped and recirculated through a reaction system to achieve efficient mixing, reaction control, and improved reaction kinetics; the recirculating reactor method where a reaction mixture is circulated within a reactor or reaction vessel to maintain optimal reaction conditions and facilitate thorough mixing; the recirculating solvent method where a solvent or reaction medium is continuously recirculated to maintain a desired reaction environment, solute concentration, and temperature control; and the recirculation of byproducts method where byproducts or undesired reaction components are continuously removed, separated, and recirculated to improve reaction selectivity and overall efficiency.

It is meant by "batch mode", a method according to which a chemical reaction is carried out by combining all reactants in a single vessel or batch reactor, allowing the reaction to proceed to completion or a desired endpoint without continuous flow or recirculation. In batch mode, the reactants are added to the reactor vessel as a discrete batch, and the reaction progresses under the specified conditions, such as temperature, pressure, and time. The reaction mixture is typically agitated or stirred to facilitate mixing and promote reaction kinetics.

It is meant by "flow mode", a method according to which a chemical reaction is conducted in a continuous flow system, where reactants are continuously pumped into the system and mixed, allowing the reaction to proceed in a controlled manner. The concept of 'flow mode' encompasses the continuous flow approach in which reactions occur in a flowing stream of reactants.

The invention relates in a third aspect to a process for the synthesis of a reaction intermediate of general formula (III) comprising a step of reacting a substituted or unsubstituted thianthrene with a compound of formula (IV): 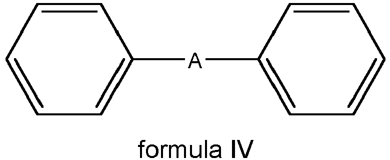 in which
- A is defined as mentioned above
in the presence of an acid and an anhydride, in at least one organic solvent.

It is meant by "a compound of formula (IV)", the compound PF-1022A previously mentioned.

Advantageously, the at least one organic solvent used for the synthesis of a reaction intermediate of general formula (III) may be selected from acetonitrile, acetic acid (AcOH) and DCM and mixtures thereof.

Advantageously, the at least one organic solvent used for the synthesis of a reaction intermediate of general formula (III) may be carried out in the presence of an acid, preferably selected from triflic acid, HBF₄OEt₂ and mixtures thereof.

Advantageously, the at least one organic solvent used for the synthesis of a reaction intermediate of general formula (III) may be carried out in the presence of an anhydride, preferably trifluoroacetic anhydride. Advantageously, the at least one organic solvent used for the synthesis of a reaction intermediate of general formula (III) may be carried out at a temperature from -30°C to 25 °C, preferably from 0 to 6°C or at -20 °C.

Advantageously, the at least one organic solvent used for the synthesis of a reaction intermediate of general formula (III) may be carried out over a period of 4 to 24 hours, preferably 4 to 16 hours and more preferably 4 hours.

The invention relates in another aspect to a process for the synthesis of emodepside comprising the following step:
- step A: reaction of a substituted or unsubstituted thianthrene with a compound of general formula (IV) in the presence an acid and an anhydride, in at least one organic solvent and obtention of a reaction intermediate of general formula (III);
- step B: reaction of a compound comprising an aliphatic or aromatic borate comprising 6 or fewer carbon atoms with the reaction intermediate of general formula (III), obtained in step A, in the presence of at least one solvent and obtention of a reaction intermediate of general formula (II);
- step C: reaction of morpholine with the reaction intermediate of general formula (II), obtained in step B, in the presence of a catalyst, in at least one organic solvent and obtention of emodepside of general formula (I).

The invention relates in another aspect to a reaction intermediate of general formula (II), wherein R¹ and A are as defined above.

The invention relates in another aspect to a reaction intermediate of general formula (III), wherein R² and A are as defined above.

The invention relates in another aspect to a use of a reaction intermediate of general formula (II) according to the invention, in a process for the synthesis of emodepside.

The invention relates in another aspect to a use of a reaction intermediate of general formula (III) according to the invention, in a process for the synthesis of emodepside.

### EXAMPLE

It is meant by "CMPE", the compound methoxycyclopentane.

### General information:

Analytical UPLC and molecular weight determinations were performed on an UPLC-ESI-MS instrument equipped with a UV-VIS (Nexera UHPLC system LC40) and a MS detector (LCMS8045 triple quadrupole Shimadzu) using a reverse phase column (Eclipse Plus C18^{®}, C18, 1.8 µm, 50 × 4.6 mm). Solvent A is Water + 0.1 % formic acid and Solvent B is acetonitrile. The total analysis time is fixed at 16 min at a flow rate of 1 L/min. The injection volume is fixed at 10 µL. The temperature of the column was fixed at 40 ° C.

For 5 min analyses: The gradient starts by equilibrating the column for 0.5 min with 5% B. The amount of B is subsequently increased to 95% for 3 min before being held isocratically for 0.5 min at 95% of B. The column is then equilibrated in 5% B for 1 min.

For 8 min analyses: The gradient starts by equilibrating the column for 1 min with 5% B. The amount of B is subsequently increased to 95% for 5 min before being held isocratically for 1 min at 95% of B. The column is then equilibrated in 5% B for 1 min.

For 16 min analyses: The gradient starts by equilibrating the column for 2 min with 5% B. The amount of B is subsequently increased to 95% for 10 min before being held isocratically for 2 min at 95% of B. The column is then equilibrated in 5% B for 2 min.

The separation is first analysed using a UV-VIS detector at a wavelength of 220 nm and 254 nm throughout the acquisition time. Then the separation is analysed using an ESI-Triple QUAD type mass spectrometer LCMS8045.

Ionization is then carried out in positive mode and in negative mode at the same time by switching between positive and negative mode every 0.3 sec using an electrospray source. Then, the compounds are analysed by a single quadrupole analyser in a mass range going from 100 to 1500 m/z. the scan speed was 5000 µ/second and the interface voltage at 4.5 kV. The interface and Desolvatation Line (DL) temperatures were fixed at 300 and 250 ° C, respectively. The temperature of the heat block was fixed at 400°C. Nebulizing gas flow and Drying gas were set at 3 L/min and 15 L/min respectively.

The data were then processed manually using the LabSolution software.

### Example 1: Synthesis of Thianthrene oxide

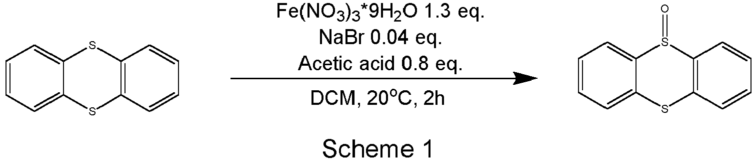

### Experimental procedure:

Thianthrene (61.14 g, 1.0 eq., 282.6 mmol) was dissolved in DCM (500 mL). Then sodium bromide (1.16 g, 0.04 eq., 11.9 mmol), iron(III) nitrate nonahydrate (148.44 g, 1.3 eq., 367.4 mmol) and acetic acid (13.58 g, 12.94 mL, 0.8 eq., 226.1 mmol) were added. The reaction mixture was stirred at 20 °C for 2 h. TLC (silica gel, 10% EtOAc in cyclohexane) showed full conversion.

### Work-up:

Water (400 mL) was added, and the phases were separated. The organic phase was washed again with NaHCOs sat. (400 mL) followed by brine (400 mL). The organic layer was separated, dried over sodium sulfate, filtered and concentrated to dryness to afford a white solid (61.0 g, 93% yield). The crude material was used without further purification.

¹H NMR (400 MHz, DMSO) δ 7.84 (dd, J = 7.9, 1.4 Hz, 2H), 7.82 (dd, J = 7.8, 1.2 Hz, 2H), 7.67 (td, J = 7.5, 1.2 Hz, 2H), 7.57 (td, J = 7.5, 1.5 Hz, 2H).

### Example 2: Synthesis of Bis-Thianthrenation of PF-1022A

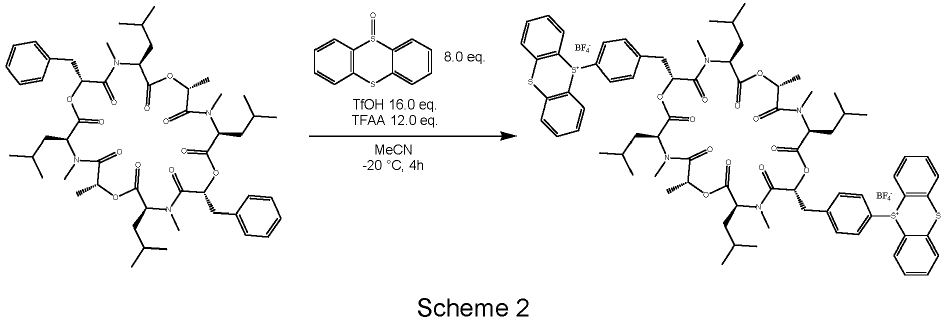

### Experimental procedure:

PF-1022A (10.0 g, 1.0 eq., 10.5 mmol) was charged in a 250 mL ATLAS reactor vessel and stirred at 400 rpm. While stirring, the starting material was dissolved in Acetonitrile (53 mL). The resulting clear homogeneous solution was cooled down to -20 °C internal temperature. Once cooled, triflic acid (25.3 g, 15.0 mL, 16.0 eq., 169.0 mmol) was added over 20 min (0.623 mL/min) using a 2.5 mL syringe pump maintaining the internal temperature below -20 °C (careful: TfOH is fuming). To this resulting clear brown homogeneous solution, was added thianthrene 5-oxide (TTO) (19.6 g, 8.0 eq., 84.3 mmol) portion-wise and the residual solid was washed down with ACN. With the addition of TTO, the reaction mixture becomes a pink suspension. Then, trifluoroacetic anhydride (26.6 g, 17.9 mL, 12.0 eq., 126.0 mmol) was added over 30 min (0.597 mL/min) using a 2.5 mL syringe pump maintaining the internal temperature below - 20 °C turning the mixture dark purple. After that, the reaction was stirred at -20 °C for 4 h.

### Work-up:

The reaction mixture in the reactor was diluted with DCM (200 mL) and slowly poured in NaHCOs sat. (400 mL) solution. Both layers were separated, and the aqueous layer was extracted again with DCM (100 mL). The combined organic layers were treated with a 5% w/w NaBF₄ solution (2 × 150 mL), dried over and Na₂SO₄, filtered and concentrated under low pressure to afford a dark brown solid.

The solid was scratched out and suspended in a pre-mixed mixture of EtOAc/CMPE 1:1 solution (200 mL). The suspension was heated at 60 °C with an oil bath and stirred for 30 min, then filtered. This operation was repeated once more. The solid was finally washed with CPME (100 mL) and dried under high vacuum to afford a brownish solid (18.00 g, 91% purity by LC-MS 254 nm, full conversion).

The EtOAc/CMPE 1:1 washings were collected and concentrated to afford a brownish solid of a mixture of crude TT/TTO (9.18 g, 38% purity by NMR).

¹H NMR (400 MHz, DMSO, rotamers) δ 8.64 - 8.51 (m, 4H), 8.10 - 8.02 (m, 4H), 7.96 - 7.90 (m, 4H), 7.89 - 7.84 (m, 4H), 7.57 - 7.48 (m, 4H), 7.23 - 7.10 (m, 4H), 5.71 - 4.29 (m, 8H), 3.79 - 3.65 (m, 1H), 3.10 - 2.63 (m, 15H), 1.84 - 1.07 (m, 15H), 0.90 - 0.79 (m, 8H), 0.79 - 0.59 (m, 19H).

LC-MS: [M/2 - 2 × BF₄]⁺ = 689.

### Example 3: TT recovery from the Bis-Thianthrenation of PF-1022A

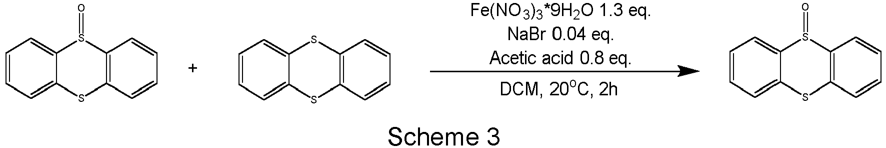

### Experimental procedure:

A mixture of thianthrene and thianthrene oxide (9.18 g, 38% purity by LC-MS 254 nm, 1.0 eq., 16.1 mmol) was dissolved in DCM (50 mL). Then sodium bromide (66 mg, 0.04 eq., 0.64 mmol), iron(III) nitrate nonahydrate (8.47 g, 1.3 eq., 21.0 mmol) and acetic acid (775 mg, 739 µL, 0.8 eq., 12.9 mmol) were added. The reaction mixture was stirred at 20 °C for 2 h. TLC (silica gel, 10% EtOAc in cyclohexane) showed full conversion.

### Work-up:

Water (100 mL) was added, and the phases were separated. The organic phase was washed again with NaHCOs sat. (100 mL) followed by brine (100 mL). The organic layer was separated, dried over sodium sulfate, filtered and concentrated to dryness to afford a beige solid (8.96 g).

¹H NMR (400 MHz, CDCl3) δ 7.88 - 7.81 (m, 4H), 7.69 (td, J = 7.5, 1.2 Hz, 2H), 7.59 (td, J = 7.5, 1.4 Hz, 2H).

### Example 4: Bis-Borylation of Bis-TT

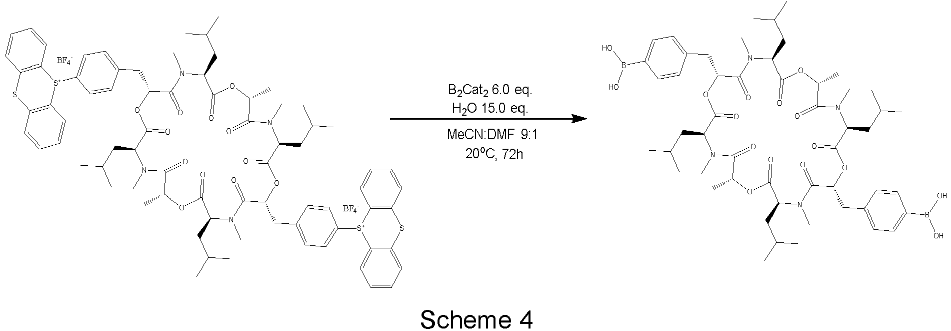

### Experimental procedure:

Bis-TT (8.63 g, 94% purity by LC-MS 220 nm, 1.0 eq., 5.22 mmol) was added in a flask followed by bis(catecholato)diborane (7.45 g, 6.0 eq., 31.3 mmol). The flask was purged with nitrogen, then dry MeCN (31 mL), dry DMF (3.48 mL) and water (1.41 g, 1.41 mL, 15.0 eq., 78.3 mmol) were added. The reaction mixture was filtered to remove the insoluble impurities and degassed using a nitrogen balloon for 5 minutes while putting it under sonication. A blue LED light (Kessil Model n° H150-BLUE; 1.5A; 34W, average wavelength = 450, maximum wavelength = 495 nm) was then turned on and the reaction mixture was stirred under blue light during 72 h.

### Work-up:

The reaction mixture was filtered to remove TT as a white powder (1.32 g, 58% recovery). Then, it was added dropwise to a stirring reaction of KHSO₄ 1 M (720 mL) and stirred for 60 min. A solid was formed. The mixture was filtered and NaHCOs sat. (150 mL) was added, stirred with the solid for 1 min to remove the acid and then filtered. Water (150 mL) was finally added and the solid was stirred for 1 min and then filtered. The solid was suspended in diisopropyl ether (DIPE) (60 mL) and the mixture was stirred at 60 °C for 30 min. The resulting solid was then dried adding toluene (25 mL) and concentrating it. This operation was repeated twice more. Then, DIPE (60 mL) was added again and the mixture was stirred at 60 °C for 30 min. The solid was dried under high vacuum to afford a white solid (5.41g, 65% purity by LCMS 200 nm, 65% yield).

The DIPE fractions were concentrated to afford a white solid containing TT (610 mg).

¹H NMR (400 MHz, DMSO, rotamers) δ 7.95 (t, J = 2.9 Hz, 4H), 7.73 (d, J = 7.8 Hz, 4H), 7.34 - 7.19 (m, 4H), 5.82 - 4.37 (m, 8H), 3.19 - 2.67 (m, 16H), 1.80 - 1.09 (m, 15H), 0.98 - 0.83 (m, 8H), 0.83 - 0.65 (m, 19H).
LC-MS: [M+H]⁺ = 1037

### Example 5: Synthesis of emodepside

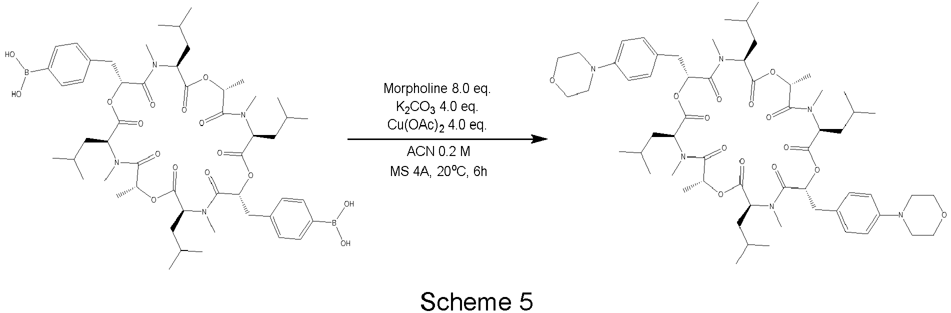

### Experimental procedure:

To a solution of Bis-B(OH)₂ (5.44 g, 78% purity including Bis-B(OH)₂ , Bis-B(OH)₂-Regioisomer and Mono-B(OH)₂, 1.0 eq., 4.09 mmol) in MeCN (21 mL) was added 4A molecular sieves (powdered, 100mg/0.3mmol), K₂CO₃ (2.26 g, 4.0 eq., 16.4 mmol), Cu(OAc)₂ (2.97 g, 4.0 eq., 16.4 mmol) and morpholine (2.85 g, 2.82 mL, 8.0 eq., 32.7 mmol). The 3-neck flask was equipped with drying tubes filled with 4A MS/cotton and the mixture was stirred at 20 °C for 16 h under air.

### Work-up:

The solvent was concentrated, CPME (100 mL) was added, and the mixture was filtered through a pad of Celite^{®} using CPME (100 mL) as eluting agent. Then, the organic phase was washed with NH₄Cl sat. (2 × 150 mL). After that, a solution of HCl 2 M (100 mL) was added and the mixture was shaken. The aqueous phase was washed again with CPME (100 mL). Then, a solution of Na₂CO₃ sat. (100 mL) was added followed by CPME (100 mL) and the mixture was extracted. The organic layer was separated, dried over Na₂SO₄ and the solvent was concentrated to afford brownish solid (987 mg, 81% purity 254 nm, 14% yield over 3 steps).

### Crystallization:

The compound (987 mg) was dissolved in the minimum amount of EtOAc, and then poured onto DIPE (50 mL) at 55 °C with continuous stirring. After 5 min, the mixture was filtered. The solid was discarded and the mother liquors were concentrated. Then, EtOH (0.4 mL) were added to the solid and the mixture was heated to 80 °C and then cooled down under continuous stirring. Once at 20 °C, the mixture was seeded and left in the fridge at 4 °C overnight. A solid appeared and it was filtered and washed with cold EtOH (2 mL). The purity was improved to 95% by HPLC.

¹H NMR (400 MHz, DMSO) δ 7.32 - 7.05 (m, 4H), 6.96 - 6.75 (m, 4H), 5.82 - 4.28 (m, 8H), 3.77 - 3.65 (m, 8H), 3.11 - 3.00 (m, 8H), 3.01 - 2.62 (m, 16H), 1.80 - 1.16 (m, 15H), 1.02 - 0.62 (m, 27H).
LC-MS: [M+H]⁺ = 1119

### Example 6:

The process resulting from the sequence of examples 2, 4 and 5 has been compared with prior art emodepside synthesis processes. Table I below shows the results of this comparison, and in particular the following criteria: number of steps, Overall yield, presence or absence of a chromatography step and use or absence of precious metals.

**Table 1: Comparison of synthesis processes**

| | Number of steps | Overall Yield | Chromatography | Precious metals |
|---|---|---|---|---|
| Examples 2, 4 and 5 | 3 | 14% | no | none |
| Process according to WO 2019091975 [1] | 13 LLS (16 total) | 4.3% | no | Pd, Ru |
| Process according to WO 2019040585 [2] | 3 | ND | yes | Pd |
| Process according to WO 2019040589 [3] | ND | <10% | yes | Pd |

### Conclusion

The process according to the invention allows to access to Emodepside from PF-1022A in few steps (3 steps), while having a great overall yield and in adequacy with a practice of green chemistry. Moreover, this process is a chromatography-free synthesis and it is precious metal-free.

### List of references

1. WO 2019091975
2. WO 2019040585
3. WO 2019040589
4. Eur. J. Org. Chem. 2012, 1546-1553.

## Claims

1. Process for the synthesis of emodepside 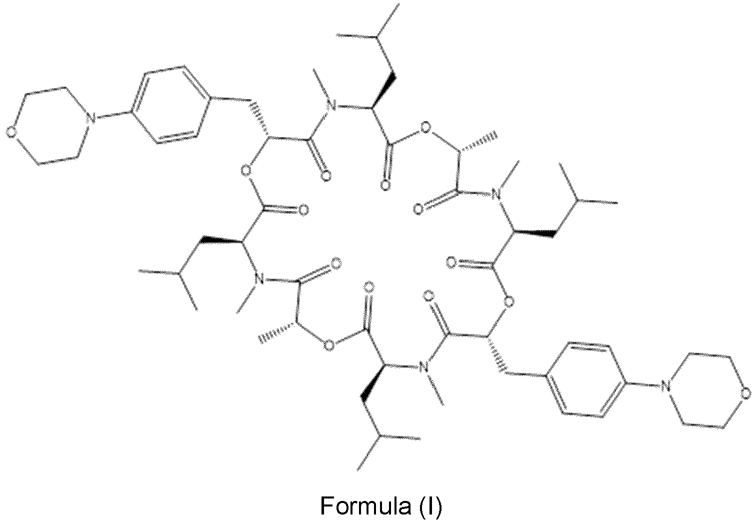 comprising a step of reacting morpholine with a reaction intermediate of formula (II): 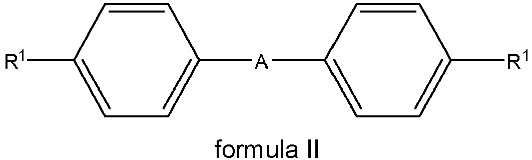 in which,
- R¹ is an aliphatic or aromatic borate group comprising 6 or fewer carbon atoms;
- A is 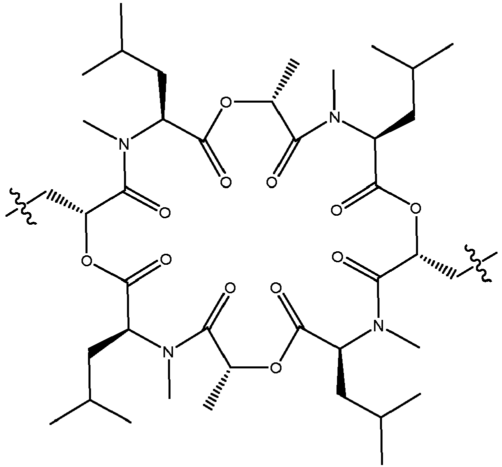 in the presence of a catalyst in at least one organic solvent.

2. The process according to claim 1, wherein the reaction is carried out in the presence of an acid or a base catalyst.

3. The process according to any of the preceding claims, wherein the reaction is carried out at a temperature from 15 to 80 °C.

4. Process for the synthesis of a reaction intermediate of general formula (II) as defined in claim 1 comprising a step of reacting a compound comprising an aliphatic or aromatic borate comprising 6 or fewer carbon atoms with a reaction intermediate of formula (III): 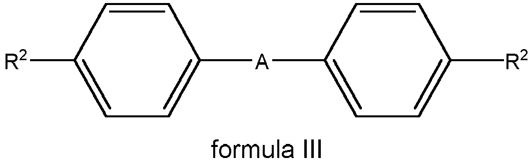 in which
- R² is a substituted or unsubstituted thianthrene group;
- A is defined as in previous claims;
in the presence of at least one solvent.

5. The process according to claim 4, wherein the process is carried out at a temperature from 15 to 80 °C.

6. The process according to any of the claims 4 to 5, further comprise the following sub-step:
a) mixing the compound comprising an aliphatic or aromatic borate comprising 6 or fewer carbon atoms with the reaction intermediate of formula (III);
b) optional introduction of a photocatalyst selected from Tris(2,2'-bipyridine)ruthenium(II) hexafluorophosphate (Ru(bpy)₃(PF₆)₂) and 2,4,5,6-tetra(carbazol-9-yl)benzene-1,3-dicarbonitrile (4czIPN);
c) irradiation of a loop of polyethylene (PE) tubing with a LED light;
d) recirculating the solution obtained in sub-step c) in the loop; and
the reaction being executed in a batch mode or a flow mode.

7. Process for the synthesis of a reaction intermediate of general formula (III) as defined in claim 4 comprising a step of reacting a substituted or unsubstituted thianthrene with a compound of formula (IV): 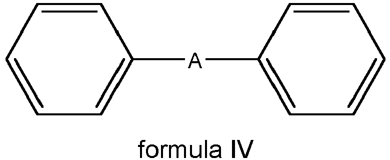 in which
- A is defined as in previous claims
in the presence of an acid and an anhydride, in at least one organic solvent.

8. The process according to claim 7, wherein the acid is selected from triflic acid, HBF₄OEt₂ and mixtures thereof.

9. The process according to any of the claims 7 to 8, wherein the process is carried out at a temperature from -30°C to 25 °C.

10. Process for the synthesis of emodepside comprising the following step :
- step A: reaction of a substituted or unsubstituted thianthrene with a compound of general formula (IV) in the presence of an acid and an anhydride, in at least one organic solvent and obtention of a reaction intermediate of general formula (III);
- step B: reaction of a compound comprising an aliphatic or aromatic borate comprising 6 or fewer carbon atoms with a reaction intermediate of general formula (III), obtained in step A, in the presence of at least one solvent and obtention of a reaction intermediate of general formula (II);
- step C: reaction of morpholine with a reaction intermediate of general formula (II), obtained in step B, in the presence of a catalyst, in at least one organic solvent and obtention of emodepside of general formula (I).

11. A reaction intermediate of general formula (II), wherein R¹ and A are as defined in claim 1.

12. A reaction intermediate of general formula (III), wherein R² and A are as defined in claim 4.

13. A use of a reaction intermediate of general formula (II) according to claim 11, in a process for the synthesis of emodepside.

14. A use of a reaction intermediate of general formula (III) according to claim 12, in a process for the synthesis of emodepside.
